# EUROPÄISCHE PATENTANMELDUNG

(11) **EP 1 992 606 A1**
(43) Veröffentlichungstag der Anmeldung: **19.11.2008**
(21) Anmeldenummer: 07108215.0
(22) Anmeldetag: 15.05.2007
(51) Int. Cl.: C07C 43/15, C07D 317/12, C07D 319/06, C07D 321/06, C11B 9/00

(54) **Alkenacetale und ihre Verwendung als Riechstoffe**

(71) Anmelder: Symrise GmbH & Co. KG, 37603 Holzminden (DE)
(72) Erfinder: Dilk, Erich, 37603, Holzminden (DE)
(74) Vertreter: Eisenführ, Speiser & Partner

(57) **Zusammenfassung**

Die vorliegende Erfindung betrifft ein Acetal der Formel (Ia), (Ib), (Ic) oder (Id) 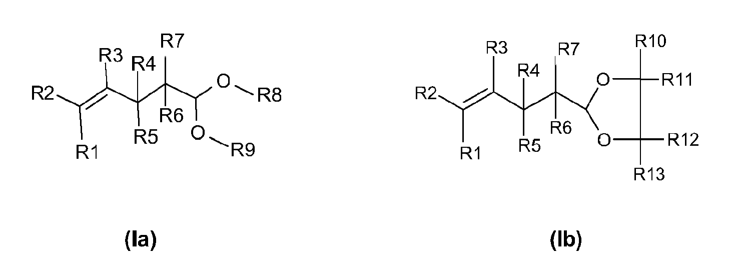 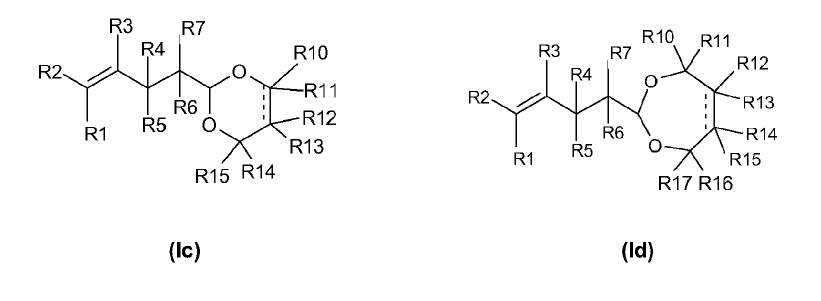 wobei
R1, R2, R3, R4, R5, R6, R7, R8, R9, R10, R11, R12, R13, R14, R15, R16 und R17 jeweils unabhängig voneinander Wasserstoff, Alkyl mit 1 bis 8 C-Atomen, Alkenyl mit 2 bis 8 C-Atomen oder Alkinyl mit 2 bis 8 C-Atomen bedeuten,
wobei am Ort einer gestrichelt dargestellten Linie zwischen zwei C-Atomen eine Einfachbindung oder Doppelbindung vorliegt, wobei bei Vorliegen einer Doppelbindung in Formel (Ic) die Gruppen R11 und R12 entfallen und in Formel (Id) die Gruppen R13 und R14 entfallen,
mit der Maßgabe, dass R4 und R5 nicht gleichzeitig Wasserstoff sind,
und
wobei die Gesamtkohlenstoffatomzahl des Acetals der Formel (Ia), (Ib), (Ic) und (Id) maximal 18, vorzugsweise maximal 14, beträgt.

## Beschreibung

Die vorliegende Erfindung betrifft neue Acetale, die sich von 4-Pentenalen ableiten, und deren Verwendung als Riechstoffe.

Wegen der im allgemeinen unzureichenden Verfügbarkeit vieler natürlicher Riechstoffkomponenten, der notwendigen Anpassung an wechselnde modische Geschmacksrichtungen sowie dem ständig steigenden Bedarf an neuen Riechstoffen, die allein oder in Form von Kompositionen wertvolle Duftstoffe bzw. Parfüms mit interessanten Duftnoten darstellen, besteht auch weiterhin ein Bedürfnis nach neuen Verbindungen mit wertvollen Riechstoffqualitäten.

Gegenstand der Erfindung sind offenkettige und cyclische Acetale der Formeln (Ia), (Ib), (Ic) oder (Id) 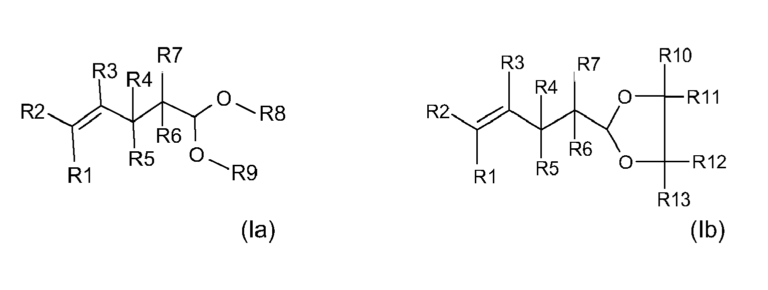 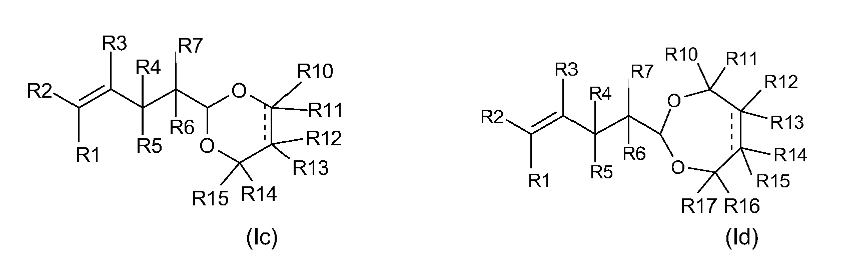 wobei
R1, R2, R3, R4, R5, R6, R7, R8, R9, R10, R11, R12, R13, R14, R15, R16 und R17 jeweils unabhängig voneinander Wasserstoff, Alkyl mit 1 bis 8 C-Atomen, Alkenyl mit 2 bis 8 C-Atomen oder Alkinyl mit 2 bis 8 C-Atomen bedeuten,
wobei am Ort einer gestrichelt dargestellten Linie zwischen zwei C-Atomen eine Einfachbindung oder Doppelbindung vorliegt, wobei bei Vorliegen einer Doppelbindung in Formel (Ic) die Gruppen R11 und R12 entfallen und in Formel (Id) die Gruppen R13 und R14 entfallen,
mit der Maßgabe, dass R4 und R5 nicht gleichzeitig Wasserstoff sind,
und
wobei die Gesamtkohlenstoffatomzahl des Acetals der Formel (Ia), (Ib), (Ic) oder (Id) maximal 18, vorzugsweise maximal 14, beträgt.

Die erfindungsgemäßen Acetale sind insbesondere zur Verwendung als Riechstoffe geeignet, die in Parfümierungen eingesetzt werden können. Die erfindungsgemäßen Verbindungen besitzen überraschenderweise blumige, grüne und teilweise auch krautige Geruchseigenschaften. Zudem weisen die erfindungsgemäßen Acetale insbesondere in alkalischen und in oxidierenden Medien eine hohe, ganz hervorragende Stabilität auf. Insbesondere wegen dieser Eigenschaften eignen sich die erfindungsgemäßen Acetale in hervorragendem Maße für die Verwendung als Riechstoffe, und zwar insbesondere, wenn sie in einer Riechstoffmischung oder einem Parfüm bzw. einem parfümierten Produkt eingesetzt werden, die bzw. das einen pH > 7 besitzt und/oder oxidierend wirkt.

EP 144 815 und DE 195 32 318 offenbaren Geruchsbeschreibungen für folgende Verbindungen, die mit den erfindungsgemäßen Substanzen strukturell eng verwandt sind, wobei hier frische und fruchtige Noten dominierend sind.
1,1-Dimethoxy-2,2,5-trimethylhex-4-en (Amarocit^{®}, Methylpampelmousse^{®}):
Frisch, fruchtig, an Grapefruit erinnernd
1,1,-Dimethoxy-2,5-dimethyl-2-vinyl-hex-4-en:
Süß, fruchtig, leicht bitterer Angeruch
2-(1,1,4-Trimethylpent-3-enyl)-4,7-dihydro-1,3-dioxepin
Frische, fruchtige und zugleich holzige Note

Die erfindungsgemäßen Acetale besitzen somit Geruchseigenschaften, die deutlich verschieden von denen der strukturell verwandten Verbindungen sind.

In erfindungsgemäß bevorzugten Acetalen bedeuten R1, R2, R3, R4, R5, R6, R7, R8, R9, R10, R11, R12, R13, R14, R15, R16 und R17 jeweils unabhängig voneinander Wasserstoff, Alkyl mit 1 bis 4 C-Atomen oder Alkenyl mit 2 bis 4 C-Atomen.

Besonders bevorzugt sind erfindungsgemäße Acetale, wobei
(i) R4 und R5 jeweils Methyl bedeuten
   oder
(ii) R1 und R2 jeweils Wasserstoff bedeuten
   Vorzugsweise bedeuten
(iii) R1 und R2 jeweils Wasserstoff sowie R4 und R5 jeweils Methyl
   Vorzugsweise bedeuten
(iv) R4, R5 und R6 jeweils Methyl
   und besonders bevorzugt bedeuten
(v) R1 und R2 jeweils Wasserstoff sowie R4, R5 und R6 jeweils Methyl.

Vorzugsweise bedeuten R1, R2 und R3 in erfindungsgemäßen Acetalen gleichzeitig Wasserstoff, wobei bevorzugt R4 und R5 gleichzeitig Methyl und ganz besonders bevorzugt auch R6 gleichzeitig Methyl bedeutet.

Besonders bevorzugt ist somit ein erfindungsgemäßes Acetal der Formel (la-x), (Ib-x), (Ic-x) oder (Id-x), 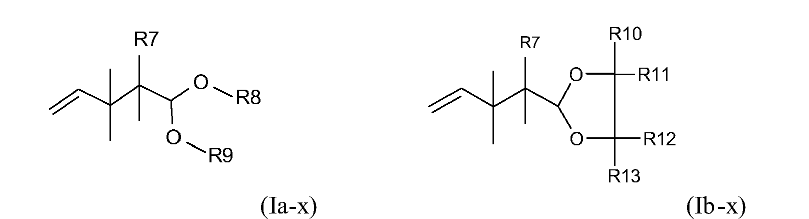 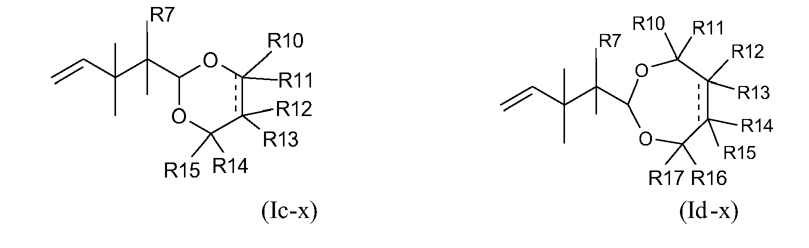 wobei
R7, R8, R9, R10, R11, R12, R13, R14, R15, R16 und R17 jeweils unabhängig voneinander Wasserstoff, Alkyl mit 1 bis 8 C-Atomen, Alkenyl mit 2 bis 8 C-Atomen oder Alkinyl mit 2 bis 8 C-Atomen bedeuten,
wobei am Ort einer gestrichelt dargestellten Linie zwischen zwei C-Atomen eine Einfachbindung oder Doppelbindung vorliegt, wobei bei Vorliegen einer Doppelbindung in Formel (Ic-x) die Gruppen R11 und R12 entfallen und in Formel (Id-x) die Gruppen R13 und R14 entfallen,und
wobei die Gesamtkohlenstoffatomzahl der Acetale der Formeln (la-x), (Ib-x), (Ic-x) und (Id-x) maximal 18, vorzugsweise maximal 14, beträgt.

Die Acetale der Formeln (la-x), (Ib-x), (Ic-x) und (Id-x) entsprechen den Acetalen der Formeln (Ia), (Ib), (Ic) und (Id), wobei jeweils gilt: R1, R2 und R3 sind jeweils Wasserstoff; R4, R5 und R6 sind jeweils Methyl.

Besonders bevorzugt sind Acetale der Formel (Ia) wie vorstehend beschrieben oder der Formel (la-x) wie vorstehend beschrieben, wobei R8 und R9 identisch sind. Vorzugsweise bedeuten R8 und R9 gleichzeitig Methyl.

Tabelle 1 zeigt besonders bevorzugten erfindungsgemäßen Acetale und deren geruchliche Eigenschaften. Diese Verbindungen wurden entsprechend den nachfolgend und im experimentellen Teil dargelegten allgemeinen Vorschriften (siehe Beispiel 1 bzw. 2) hergestellt.

**Tabelle 1 : besonders bevorzugte erfindungsgemäße Acetale**

| Verbindung | Formel | Sdp. / Kₚ | Geruchliche Beschreibung |
|---|---|---|---|
| 1 | 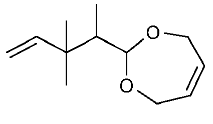 | 93°C/3,8 mbar | Blüte, Rose, Geraniol |
| 2 | 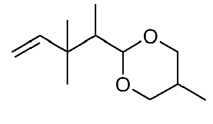 | 89°C/3,5 mbar | Blüte, Carbinol, Ozon, Grün |
| 3 | 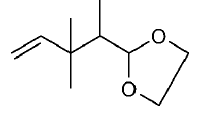 | 63°C/6,0 mbar | Rose, Blüte, Carbinol, Damascon, Kraut |
| 4 | 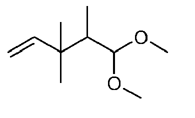 | 93°C/56 mbar | Grün, Blattgrün, Gemüse, Kraut, Rose |
| 5 | 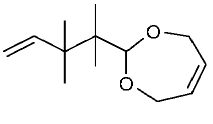 | 111°C/7,5 mbar | Agrumen, Grün, Styrolylacetat, Vetiver |
| 6 | 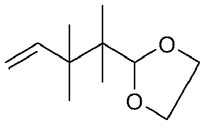 | 81°C/6,0 mbar | Frisch, Kraut, Grün |
| 7 | 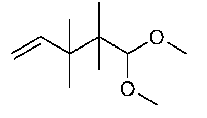 | 126°C/84 mbar | Grün, Kraut, Kampfer, Blüte, Carbinol, Rose, Geraniol |

Die erfindungsgemäßen Acetale sind neu und lassen sich nach an sich bekannten Syntheseverfahren der organischen Chemie erhalten. Gemäß einem erfindungsgemäßen Verfahren zur Herstellung eines offenkettigen Acetals der Formel (Ia) oder eines cyclischen Acetals der Formel (Ib), (Ic) oder (Id) (wie oben beschrieben, vorzugsweise in einer der vorstehend als bevorzugt bezeichneten Ausgestaltungen) wird ein 4-Pentenal bzw. 4-Pentenalderivat der Formel (P-Ald) 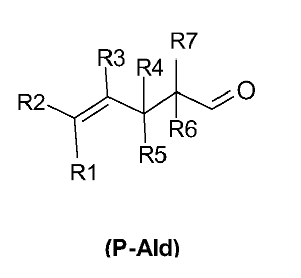
(i) mit aliphatischen Alkoholen der Formeln

   R8-OH, R9-OH

   zu dem offenkettigen Acetal der Formel (Ia) oder
(ii) mit einem 1,2-, 1,3- bzw. 1,4-Diol der Formeln 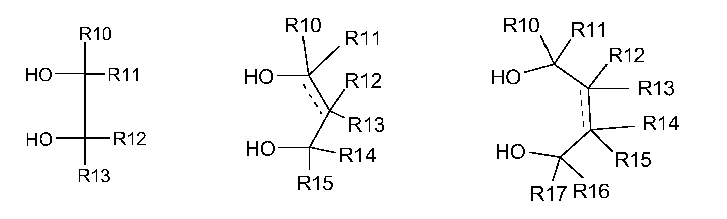 zu den cyclischen Acetalen der Formeln (Ib), (Ic) bzw. (Id)
   unter Säurekatalyse und Wasserabscheidung umgesetzt,
wobei die Gruppen R1, R2, R3, R4, R5, R6, R7, R8,. R9, R10, R11, R12, R13, R14, R15, R16 und R17 sowie die gestrichelten Linien jeweils die oben angegebene Bedeutung haben.

Es versteht sich, dass die Substituenten (Gruppen) an den 1,2-, 1,3- bzw. 1,4-Diolen festlegen, welche Bedeutung die Gruppen R10, R11, R12, R13, R14, R15, R16, R17 in den entsprechenden Acetalen der Formel (Ib), (Ic) bzw. (Id)
(vorzugsweise der Formel (Ib-x), (Ic-x) und (Id-x)) besitzen.

Das folgende Schema veranschaulicht diese vorteilhafte Acetalisierung:

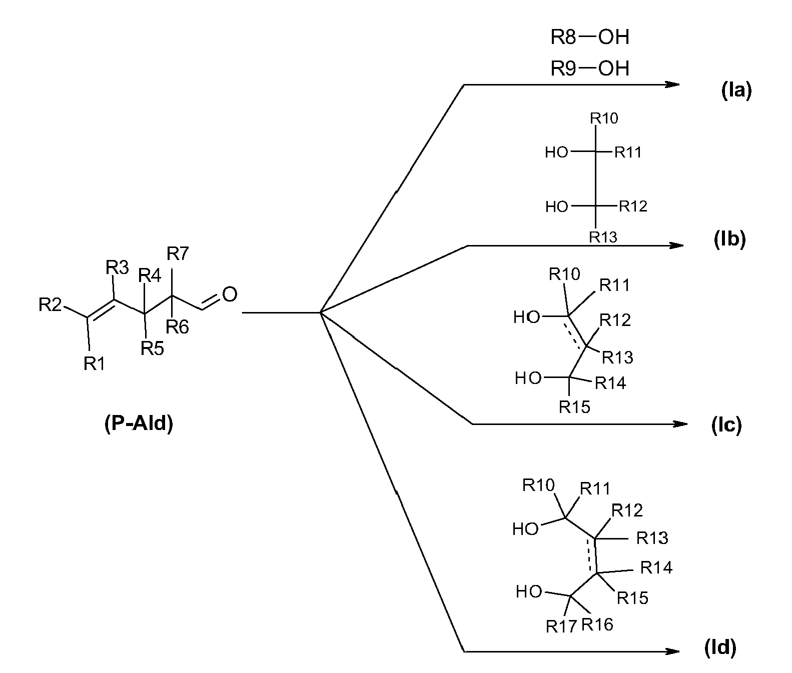

In den gezeichneten Formeln besitzen die Gruppen R1 bis R17 die oben für die Verbindungen (Ia), (Ib), (Ic), (Id) bzw. für die vorteilhaften Verbindungen (la-x), (Ib-x), (Ic-x) und (Id-x) angegebene Bedeutung haben.

Bei der genannten Reaktionsführung wird das gebildete Wasser vorteilhafterweise durch Hinzufügen eines wasserbindenden Mittels oder durch Destillation abgetrennt. Als wasserbindende und gleichzeitig acetalisierende Mittel werden bevorzugt Orthocarbonsäureester eingesetzt, insbesondere Trialkylorthoameisensäureester wie Orthoameisensäuretrimethyl- oder -triethylester. Für eine Wasserabtrennung durch Destillation wird bevorzugt eine azeotrope Destillation unter Verwendung eines Schleppmittels genutzt. Als Schleppmittel sind insbesondere inerte Lösungsmittel wie Toluol, Xylol, Cyclohexan oder n-Pentan geeignet.

Die als Edukte verwendeten 4-Pentenale bzw. deren Derivate (P-Ald) sind erhältlich analog zu J. Org. Chem. 1977, 42, 3360 durch Ruthenium-(II) katalysierte Umlagerung von Diallylethern.

Die Erfindung betrifft auch die Verwendung eines erfindungsgemäßen Acetals (wie oben definiert, insbesondere in einer der oben als bevorzugt angegebenen Ausgestaltungen) (a) als Riechstoff oder (b) zur Herstellung einer Riechstoffmischung oder eines Parfüms.

Die vorliegende Erfindung betrifft zudem Produkte umfassend
- einen Träger oder ein Substrat sowie
- eine damit in direktem Kontakt stehende sensorisch wirksame Menge eines Acetals (wie oben beschrieben, vorzugsweise wie oben als besonders bevorzugt angegeben).

Bevorzugte erfindungsgemäße Produkte sind ausgewählt aus der Gruppe bestehend aus: alkoholischen Parfüms, Körperpflegeprodukten und im Haushalt zu verwendenden Reinigungs- oder Pflegeprodukten. Dabei sind die Körperpflegeprodukte vorzugsweise ausgewählt aus der Gruppe bestehend aus Seifen, Duschgelen, Shampoos, Badezusätzen, Hautcremes, Körperlotionen und Deodorantien, und die Reinigungsmittel sind vorzugsweise ausgewählt aus der Gruppe bestehend aus Waschmitteln, Wäscheweichspülern, Raumluftverbesserern und Reinigern.

Die erfindungsgemäßen Acetale lassen sich mit anderen Riechstoffen in verschiedenen, unterschiedlichen Mengenverhältnissen zu neuartigen Parfümkompositionen kombinieren.

Beispiele für Riechstoffe, mit denen die erfindungsgemäßen Acetale vorteilhaft kombiniert werden können, finden sich z.B. in S. Arctander, Perfume and Flavor Materials, Vol. I und II, Montclair, N. J., 1969, Selbstverlag oder H. Surburg und J. Panten, Common Fragrance and Flavor Materials, 5th Ed., Wiley-VCH, Weinheim 2006.

Im einzelnen seien genannt:
Extrakte aus natürlichen Rohstoffen wie Etherische Öle, Concretes, Absolues, Resine, Resinoide, Balsame, Tinkturen wie z. B. Ambratinktur; Amyrisöl; Angelicasamenöl; Angelicawurzelöl; Anisöl; Baldrianöl; Basilikumöl; Baummoos -Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam ; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-Öl; Eucalyptusöl; Fenchelöl ; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl; Lavendel-Absolue; Lavendelöl; Lemongrasöl; Liebstocköl; Limetteöl destilliert; Limetteöl gepreßt; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-ÖI; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl, sowie Fraktionen davon, bzw. daraus isolierten Inhaltsstoffen;

Einzel-Riechstoffe aus der Gruppe
der Kohlenwasserstoffe, wie z. B. 3-Caren; α- Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan;
der aliphatischen Alkohole wie z. B.
Hexanol; Octanol; 3-Octanol; 2,6-Dimethylheptanol; 2-Methyl-2-heptanol; 2-Methyl-2-octanol; (E)-2-Hexenol; (E)- und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol;
der aliphatischen Aldehyde und deren Acetale wie z. B.
Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal; 2,6,10-Trimethyl-5,9-undecadienal; Heptanaldiethylacetal; 1,1-Dimethoxy-2,2,5-trimethyl-4-hexen; Citronellyloxyacetaldehyd;
der aliphatischen Ketone und deren Oxime wie z. B.
2-Heptanon; 2-Octanon; 3-Octanon; 2-Nonanon; 5-Methyl-3-heptanon ; 5-Methyl-3-heptanonoxim; 2,4,4,7-Tetramethyl-6-octen-3-on; 6-Methyl-5-hepten-2-on;
der aliphatischen schwefelhaltigen Verbindungen wie z. B.; 3-Methylthiohexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
der aliphatischen Nitrile wie z.B.; 2-Nonensäurenitril; 2-Tridecensäurenitril; 2,12-Tridecadiensäurenitril; 3,7-Dimethyl-2,6-octadiensäurenitril; 3,7-Dimethyl-6-octensäurenitril;
der aliphatischen Carbonsäuren und deren Ester wie z.B. (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat, ; Isoamylbutyrat; Hexylbutyrat; (E)- und (Z)-3-Hexenylisobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadienoat;
der acyclischen Terpenalkohole wie z. B. Citronellol; Geraniol; Nerol; Linalool; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate, 3-Methyl-2-butenoate;
der acyclischen Terpenaldehyde und -ketone wie z. B.; Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;
der cyclischen Terpenalkohole wie z. B. Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate, 3-Methyl-2-butenoate;
der cyclischen Terpenaldehyde und -ketone wie z. B.:; Menthon; Isomenthon ; 8-Mercaptomenthan-3-on ; Carvon; Campher; Fenchon; alpha-Ionon; beta-Ionon; alphan-Methylionon; beta-n-Methylionon; alpha-Isomethylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on; Nootkaton; Dihydronootkaton; alpha-Sinensal; beta-Sinensal; Acetyliertes Cedernholzöl (Methylcedrylketon);
der cyclischen Alkohole wie z.B. 4-tert.-Butylcyclohexanol ; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;
der cycloaliphatischen Alkohole wie z.B. alpha,3,3-Trimethylcyclohexylmethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
der cyclischen und cycloaliphatischen Ether wie z.B. Cineol; Cedrylmethylether; Cyclododecylmethylether; (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
der cyclischen und makrocyclischen Ketone wie z.B. ; 4-tert.-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 8-Cyclohexadecen-1-on; 9-Cycloheptadecen-1-on; Cyclopentadecanon; Cyclohexadecanon; der cycloaliphatischen Aldehyde wie z.B. ; 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
der cycloaliphatischen Ketone wie z. B. ; 1-(3,3-Dimethylcyclohexyl)-4-penten-1-on; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
der Ester cyclischer Alkohole wie z.B.; 2-tert-Butylcyclohexylacetat; 4-tert-Butylcyclohexylacetat; 2-tert-Pentylcyclohexylacetat; 4-tert-Pentylcyclohexylacetat; Decahydro-2-naphthylacetat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methanooctahydro-5, bzw. 6-indenylacetat;
der Ester cycloaliphatischer Carbonsäuren wie z. B. ; Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
der araliphatischen Alkohole wie z.B. ; Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-l-ol; 4-Methoxybenzylalkohol; 1-(4-Isopropylphenyl)ethanol;
der Ester von araliphatischen Alkoholen und aliphatischen Carbonsäuren wie z.B. ; Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat;
der araliphatischen Ether wie z.B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxane; 4,4a,5,9b-Tetrahydroindeno[1,2-d]-m-dioxin; 4,4a,5,9b-Tetrahydro-2,4-dimethylindeno[1,2-d]-m-dioxin; der aromatischen und araliphatischen Aldehyde wie z. B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
der aromatischen und araliphatischen Ketone wie z.B. Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon; Benzophenon; 1,1,2,3,3,6-Hexamethyl-5-indanylmethylketon; 6-tert.-Butyl-1,1-dimethyl-4-indanylmethylketon; 1-[2,3-dihydro-1,1,2,6-tetramethyl-3-(1-methylethyl)-1H-5-indenyl]ethanon; 5',6',7',8'-Tetrahydro-3',5',5',6',8',8'-hexamethyl-2-acetonaphthon;
der aromatischen und araliphatischen Carbonsäuren und deren Ester wie z.B. Benzoesäure; Phenylessigsäure; Methylbenzoat; Ethylbenzoat; Hexylbenzoat; Benzylbenzoat; Methylphenylacetat; Ethylphenylacetat; Geranylphenylacetat; Phenylethylphenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen wie z.B. 2,4,6-Trinitro-1,3-dimethyl-5-tert.-butylbenzol; 3,5-Dinitro-2,6-dimethyl-4-tert.-butylacetophenon; Zimtsäurenitril; 5-Phenyl-3-methyl-2-pentensäurenitril; 5-Phenyl-3-methylpentansäurenitril; Methylanthranilat; Methy-N-methylanthranilat; Schiff'sche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-Isopropylchinolin; 6-Isobutylchinolin; 6-sec.-Butylchinolin; Indol; Skatol; 2-Methoxy-3-isopropylpyrazin; 2-Isobutyl-3-methoxypyrazin;
der Phenole, Phenylether und Phenylester wie z.B. Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin.;

In Parfümkompositionen beträgt die eingesetzte Gesamtmenge an erfindungsgemäßen Acetalen vorteilhafterweise 0,05 bis 25 Gew.-%, vorzugsweise 0,1 bis 15 Gew.-%, bezogen auf das Gesamtgewicht der Parfümkomposition.

Es wurde ferner gemäß einem weiteren Aspekt der Erfindung gefunden, dass sich die erfindungsgemäßen Acetale, insbesondere in geringen Konzentrationen, hervorragend zum Modifizieren und/oder Verstärken eines Geruchs eignen, d.h. dass die Acetale insbesondere als sogenannte Booster oder Enhancer fungieren können.

In einem weiteren Aspekt betrifft die vorliegende Erfindung daher die Verwendung der erfindungsgemäßen Acetale zum Modifizieren und/oder Verstärken eines Geruchs, insbesondere von blumigen Noten, speziell von Noten der Richtungen Rose, Geranium, Blüte und Damascon. Diese geruchlichen Effekte sind beispielsweise in Beispiel 3 dargelegt; vergleiche aber auch die obige Tabelle 1 betreffend besonders bevorzugte erfindungsgemäße Acetale.

Sofern die erfindungsgemäßen Acetale hauptsächlich eingesetzt werden um in einer Riechstoffkomposition bestimmte Noten zu verstärken, insbesondere blumige Noten, ist der Anteil der erfindungsgemäßen Acetale vorzugsweise recht niedrig und vorzugsweise im Bereich von 0,05 bis 2 Gew.-%, bevorzugt im Bereich von 0,1 bis 1 Gew.-%, bezogen auf die Gesamtmenge der Riechstoffkomposition. Sofern innerhalb der bevorzugten Konzentrationsbereiche eine vergleichsweise niedrige Konzentration gewählt wird, kommt es in Abhängigkeit von den weiteren Komponenten der jeweiligen Komposition in manchen Fällen noch nicht zu der Vermittlung der oben angegebenen Eigengeruchsnoten.

Erfindungsgemäße Acetale enthaltende Parfümöle können in flüssiger Form, unverdünnt oder mit einem Lösungmittel verdünnt für Parfümierungen eingesetzt werden. Geeignete Lösungsmittel hierfür sind z.B. Ethanol, Isopropanol, Diethylenglycolmonoethylether, Glycerin, Propylenglycol, 1,2-Butylenglycol, Dipropylenglycol, Diethylphthalat, Triethylcitrat, Isopropylmyristat usw.

Des weiteren können erfindungsgemäße Acetale enthaltende Parfümöle an einem Trägerstoff adsorbiert sein, der sowohl für eine feine Verteilung der Riechstoffe im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer und Cellulose-basierende Stoffe sein.

Erfindungsgemäße Acetale enthaltende Parfümöle können auch mikroverkapselt, sprühgetrocknet, als Einschluß-Komplexe oder als Extrusions-Produkte vorliegen und in dieser Form dem zu parfümierenden Produkt hinzugefügt werden.

Gegebenenfalls können die Eigenschaften der derart modifizierten Parfümöle durch sog "Coaten" mit geeigneten Materialien im Hinblick auf eine gezieltere Duftfreisetzung weiter optimiert werden, wozu vorzugsweise wachsartige Kunststoffe wie z.B. Polyvinylalkohol verwendet werden.

Die Mikroverkapselung der Parfümöle kann beispielsweise durch das sogenannte Koazervationsverfahren mit Hilfe von Kapselmaterialien z.B. aus polyurethan-artigen Stoffen oder Weichgelatine, erfolgen. Die sprühgetrockneten Parfümöle können beispielsweise durch Sprühtrocknung einer das Parfümöl enthaltenden Emulsion, bzw. Dispersion hergestellt werden, wobei als Trägerstoffe modifizierte Stärken, Proteine, Dextrin und pflanzliche Gummen verwendet werden können. Einschluß-Komplexe können z.B. durch Eintragen von Dispersionen von dem Parfümöl und Cyclodextrinen oder Harnstoffderivaten in ein geeignetes Lösungsmittel, z.B. Wasser, hergestellt werden. Extrusions-Produkte können durch Verschmelzen der Parfümöle mit einem geeigneten wachsartigen Stoff und durch Extrusion mit nachfolgender Erstarrung, ggf. in einem geeigneten Lösungsmittel, z.B. Isopropanol, erfolgen.

Erfindungsgemäße Acetale enthaltende Parfümöle können in konzentrierter Form, in Lösungen oder in oben beschriebener modifizierter Form verwendet werden für die Herstellung von z.B. Parfüm-Extraits, Eau de Parfums, Eau de Toilettes, Rasierwässer, Eau de Colognes, Pre-shave-Produkte, Splash-Colognes und parfümierten Erfrischungstüchern sowie die Parfümierung von sauren, alkalischen und neutralen Reinigungsmitteln, wie z.B. Fußbodenreinigern, Fensterglasreinigern, Geschirrspülmittel, Bad- und Sanitärreinigern, Scheuermilch, festen und flüssigen WC-Reinigern, pulver- und schaumförmigen Teppichreinigern, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendesinfektionsmitteln sowie von Luftverbesserern in flüssiger, gelartiger oder auf einem festen Träger aufgebrachter Form, Aerosolsprays, Wachsen und Polituren wie Möbelpolituren, Fußbodenwachsen, Schuhcremes sowie Körperpflegemitteln wie z.B. festen und flüssigen Seifen, Duschgelen, Shampoos, Rasierseifen, Rasierschäumen, Badeölen, kosmetischen Emulsionen vom Öl-in-Wasser-, vom Wasser-in-Öl- und vom Wasser-in-Öl-in-Wasser-Typ wie z.B. Hautcremes- und -lotionen, Gesichtscremes und -lotionen, Sonnenschutzcremes-und -lotionen, After-sun-cremes und -lotionen, Handcremes und -lotionen, Fußcremes und -lotionen, Enthaarungscremes und -lotionen, After-shave-Cremes und -lotionen, Bräunungscremes und -lotionen, Haarpflegeprodukten wie z.B. Haarsprays, Haargelen, festigen Haarlotionen, Haarspülungen, permanenten und semipermanenten Haarfärbemitteln, Haarverformungsmitteln wie kaltwellen und Haarglättungsmitteln, Haarwässern, Haarcremes und -lotionen, Deodorantien und Antiperspirantien wie z.B. Achselsprays, Roll-ons, Deosticks, Deocremes, Produkten der dekorativen Kosmetik wie z.B. Lidschatten, Nagellacke, Make-ups, Lippenstifte, Mascara sowie von Kerzen, Lampenölen, Räucherstäbchen, Insektiziden, Repellentien, Treibstoffen.

Die nachfolgenden Beispiele sollen die Erfindung (in ihren verschiedenen Aspekten) näher erläutern. Sofern nicht anders angegeben. beziehen sich alle Angaben auf das Gewicht.

### Beispiel 1: Allgemeine Herstellvorschrift der offenkettigen Acetale der Formel (Ia)

1,5 Mol aliphatischer Alkohol (bzw. Mischung zweier aliphatischer Alkohole), 0,55 mol Trialkylorthoameisensäureester und 0,5 g p-Toluolsulfonsäure werden in einem 500 mL-Dreihalskolben, versehen mit Rückflußkühler, Rührer und Tropftrichter, vorgelegt. Hierzu dosiert man bei Raumtemperatur innerhalb von 1 Stunde 0,5 mol des entsprechenden 4-Pentenals bzw. des 4-Pentenalderivats (P-Ald). Zur Vervollständigung der Reaktion wird 2 Stunden unter Rückfluß erhitzt. Man fügt 2 g Natriumcarbonat hinzu und destilliert zunächst unter Normaldruck die Leichtsieder ab und fraktioniert danach unter vermindertem Druck.

### Beispiel 2: Allgemeine Herstellvorschrift der cyclischen Acetale der Formeln (Ib), (Ic), (Id)

1 mol des entsprechenden 4-Pentenals bzw. des 4-Pentenalderivats (P-Ald), 1,5 mol 1,2-, 1,3- oder 1,4-Diol, 0,1 g p- Toluolsulfonsäure und 300 mL Toluol werden in einer 1 L-Rührapparatur 2-3 Stunden unter Verwendung eines Wasserabscheiders am Rückfluß erhitzt. Nach beendeter Wasserabspaltung wird die abgekühlte Reaktionsmischung mit 5%-iger Natriumhydrogencarbonat-Lösung gewaschen und die Phasen getrennt. Die organische Phase wird unter Zusatz von 2 g Natriumcarbonat zunächst unter Normaldruck und danach unter vermindertem Druck destilliert.

### Beispiel 3: Erfindungsgemäße Riechstoffkomposition im Vergleich mit einer Basiskomposition

### Basiskomposition

| Riechstoff | Gew.-Teile |
|---|---|
| Aldehyd C11 Iso | 3 |
| Dihydromyrcenol | 5 |
| Methylnaphthylketon beta, 10%ig in TEC | 2 |
| D-Limonen | 1 |
| Cyclamenaldehyd | 4 |
| Geraniumöl | 20 |
| 2-Phenoxyethylisobutyrat | 30 |
| Phenylethylalkohol | 500 |
| Citronellol | 100 |
| Geraniol | 100 |
| 2-Methyl-5-phenylpentanol | 1 |
| Geranylacetat | 20 |
| Damascenon 1%ig in DPG | 15 |
| Damascon Beta 10%ig in TEC | 5 |
| Benzophenon | 12 |
| Diphenyloxid | 5 |
| 2-Methylnonensäuremethylester 10%ig in TEC | 3 |
| Vanillin 10% ig in TEC | 1 |
| Phenylethylcinnamat | 16 |
| Phenylessigsäure 10%ig in TEC | 2 |
| Isobornylcyclohexanol | 10 |
| Triethylcitrat | 145 |
| Summe | 1000 |

| | |
|---|---|
| TEC = Triethylcitrat; DPG = Dipropylenglycol | |

Durch die Zugabe von nur 0,2 Gew.-% von Verbindung 3 (siehe Tabelle 1, oben) zu der angegebenen Basiskomposition wirkt diese rosiger, weicher und etwas damasconiger, wobei die Blütennote insgesamt verstärkt wird.

### Beispiel 4: Shampoo

Die Parfümölkomposition aus Beispiel 3 (nach Zusatz des erfindungsgemäßen Acetals) wurde in einer Dosierung von 0,5 Gew.-% in eine Shampoo-Grundmasse folgender Zusammensetzung eingearbeitet:

| | |
|---|---|
| Natriumlaurylethersulfat (z.B. Texapon NSO, Fa. Cognis Deutschland GmbH) | 12% |
| Cocamidopropylbetain (z.B. Dehyton K, Fa. Cognis Deutschland GmbH) | 2% |
| Natriumchlorid | 1,4% |
| Citronensäure | 1,3% |
| | |
| Phenoxyethanol, Methyl-, Ethyl-, Butyl-,und Propylparaben | 0,5% |
| Wasser | 82,8% |

Hieraus wurden anschließend 100 mL einer wässrigen Shampoo-Lösung hergestellt (pH-Wert ca. 6,7). In dieser Shampoo-Lösung wurden 2 Haarsträhnchen gemeinsam für 2 Minuten gewaschen und anschließend 20 Sekunden unter fließendem handwarmen Wasser gespült. Eine Haarsträhne wurde nass in Aluminiumfolie eingepackt und die zweite Haarsträhne mit einem Fön getrocknet. Beide Haarsträhnen wurden von einem Panel geruchlich beurteilt.

### Beispiel 5: Waschpulver

Die Parfümölkomposition aus Beispiel 3 (nach Zusatz des erfindungsgemäßen Acetals) wurde in einer Dosierung von 0,4 Gew.-% in eine Waschpulver-Grundmasse der folgenden Rezeptur eingearbeitet:

| | |
|---|---|
| Lineares Na-Alkylbenzolsulfonat | 8,8 % |
| Ethoxylierter Fettalkohol C12-18 (7 EO) | 4,7 % |
| Na-Seife | 3,2 % |
| Entschäumer | |
| DOW CORNING(R) 2-4248S POWDERED ANTIFOAM, | |
| Silikonöl auf Zeolith als Trägermaterial | 3,9 % |
| Zeolith 4A | 28,3 % |
| Na-Carbonat Na-Salz eines Copolymers aus Acryl- | 11,6 % |
| und Maleinsäure (Sokalan CP5) | 2,4 % |
| Na-Silikat | 3,0 % |
| Carboxymethylcellulose | 1,2 % |
| Dequest 2066 | 2,8 % |
| ([[(Phosphonomethyl)imino]bis[(ethylennitrilo)bis (methylen)]]tetrakis-phosphonsäure, Natriumsalz) | |
| Optischer Aufheller | 0,2 % |
| Na-Sulfat | 6,5 % |
| Protease | 0,4 % |
| Natriumperborattetrahydrat | 22,0 % |
| TAED | 1,0 % |

Zwei Stofflappen wurden mit 370 g einer 1%-igen wässrigen Waschpulverlauge (der pH-Wert der Waschpulverlauge liegt deutlich im basischen Bereich) in einer Linetest-Maschine im Hauptwaschgang 45 Minuten bei 60°C gewaschen. Die Lappen wurden zunächst 5 Minuten mit kaltem Wasser gespült, ausgewrungen und anschließend 20 Sekunden geschleudert. Ein Lappen wurde nass eingeschweißt, und einer zum Trocknen aufgehängt. Anschließend wurden beide Lappen durch ein Panel geruchlich beurteilt.

## Patentansprüche

1. Acetal der Formel (Ia), (Ib), (Ic) oder (Id) 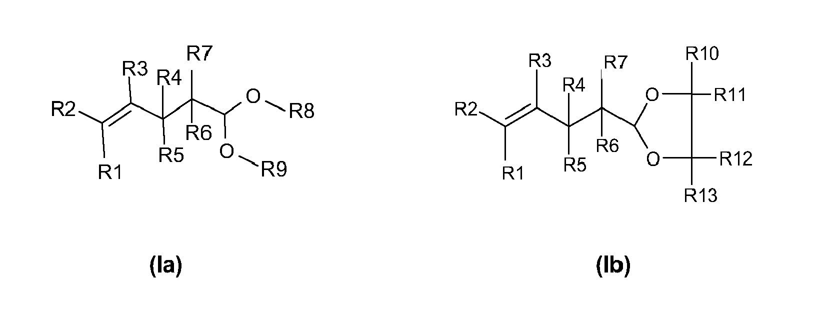 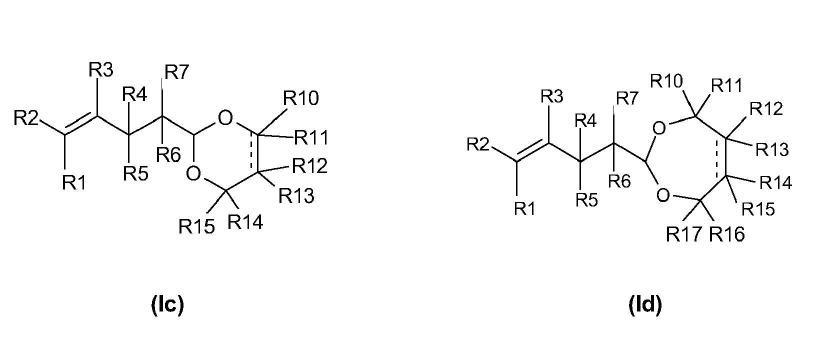 wobei
R1, R2, R3, R4, R5, R6, R7, R8, R9, R10, R11, R12, R13, R14, R15, R16 und R17 jeweils unabhängig voneinander Wasserstoff, Alkyl mit 1 bis 8 C-Atomen, Alkenyl mit 2 bis 8 C-Atomen oder Alkinyl mit 2 bis 8 C-Atomen bedeuten,
wobei am Ort einer gestrichelt dargestellten Linie zwischen zwei C-Atomen eine Einfachbindung oder Doppelbindung vorliegt, wobei bei Vorliegen einer Doppelbindung in Formel (Ic) die Gruppen R11 und R12 entfallen und in Formel (Id) die Gruppen R13 und R14 entfallen,
mit der Maßgabe, dass R4 und R5 nicht gleichzeitig Wasserstoff sind,
und
wobei die Gesamtkohlenstoffatomzahl des Acetals der Formel (Ia), (Ib), (Ic) und (Id) maximal 18, vorzugsweise maximal 14, beträgt.

2. Acetal nach Anspruch 1, wobei R1, R2, R3, R4, R5, R6, R7, R8,. R9, R10, R11, R12, R13, R14, R15, R16 und R17 jeweils unabhängig voneinander Wasserstoff, Alkyl mit 1 bis 4 C-Atomen oder Alkenyl mit 2 bis 4 C-Atomen bedeuten.

3. Acetal nach einem der vorangehenden Ansprüche, wobei
(i) R4 und R5 jeweils Methyl bedeuten
oder
(ii) R1 und R2 jeweils Wasserstoff bedeuten
oder vorzugsweise
(iii) R1 und R2 jeweils Wasserstoff sowie R4 und R5 jeweils Methyl bedeuten
oder vorzugsweise
(iv) R4, R5 und R6 jeweils Methyl bedeuten
oder besonders bevorzugt
(v) R1 und R2 jeweils Wasserstoff sowie R4, R5 und R6 jeweils Methyl bedeuten.

4. Acetal der Formel (la-x), (Ib-x), (Ic-x) oder (Id-x), 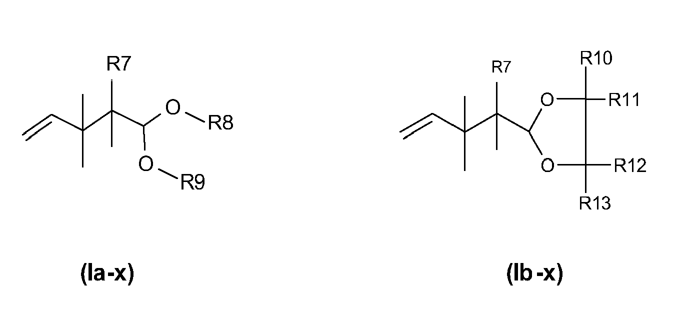 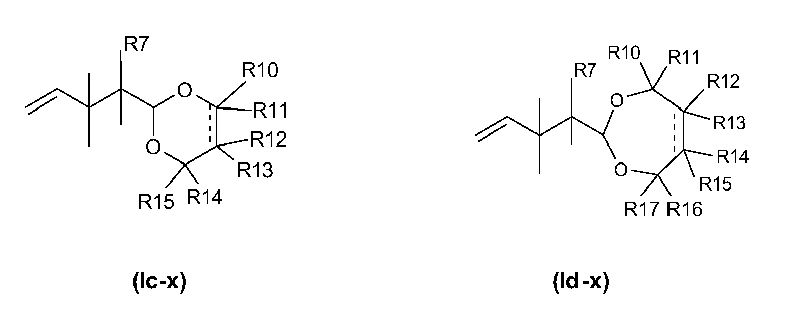 wobei
R7, R8,. R9, R10, R11, R12, R13, R14, R15, R16 und R17 jeweils unabhängig voneinander Wasserstoff, Alkyl mit 1 bis 8 C-Atomen, Alkenyl mit 2 bis 8 C-Atomen oder Alkinyl mit 2 bis 8 C-Atomen bedeuten,
wobei am Ort einer gestrichelt dargestellten Linie zwischen zwei C-Atomen eine Einfachbindung oder Doppelbindung vorliegt, wobei bei Vorliegen einer Doppelbindung in Formel (Ic-x) die Gruppen R11 und R12 entfallen und in Formel (Id-x) die Gruppen R13 und R14 entfallen,und
wobei die Gesamtkohlenstoffatomzahl der Acetale der Formeln (la-x), (Ib-x), (Ic-x) und (Id-x) maximal 18, vorzugsweise maximal 14, beträgt.

5. Acetal der Formel (Ia) nach einem der vorangehenden Ansprüche 1 bis 3 oder der Formel (la-x) nach Anspruch 4, wobei R8 und R9 identisch sind.

6. Verwendung eines Acetals nach einem der Ansprüche 1 - 5
(a) als Riechstoff oder
(b) zur Herstellung einer Riechstoffmischung oder eines Parfüms.

7. Produkt, umfassend
- einen Träger oder ein Substrat sowie
- eine damit in direktem Kontakt stehende sensorisch wirksame Menge eines Acetals nach einem der Ansprüche 1-5.

8. Produkt nach Anspruch 7, ausgewählt aus der Gruppe bestehend aus: alkoholischen Parfüms, Körperpflegeprodukten und im Haushalt zu verwendenden Reinigungs- oder Pflegeprodukten.

9. Produkt nach Anspruch 8, **dadurch gekennzeichnet, dass** die Körperpflegeprodukte ausgewählt sind aus der Gruppe bestehend aus Seifen, Duschgelen, Shampoos, Badezusätzen, Hautcremes, Körperlotionen und Deodorantien, und die Reinigungsmittel ausgewählt sind aus der Gruppe bestehend aus Waschmitteln, Wäscheweichspülern, Raumluftverbesserern und Reinigern.

10. Verfahren zur Herstellung eines offenkettigen Acetals der Formel (Ia) oder eines cyclischen Acetals der Formel (Ib), (Ic) oder (Id) nach einem der Ansprüche 1-5, **dadurch gekennzeichnet, dass** ein 4-Pentenal der Formel 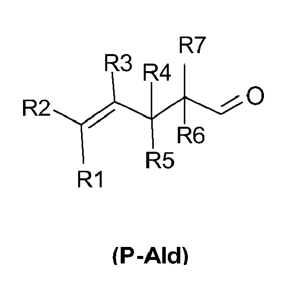
(i) mit aliphatischen Alkoholen der Formeln
R8-OH, R9-OH
zu dem offenkettigen Acetal der Formel (Ia) oder
(ii) mit einem 1,2-, 1,3- bzw. 1,4-Diol der Formel 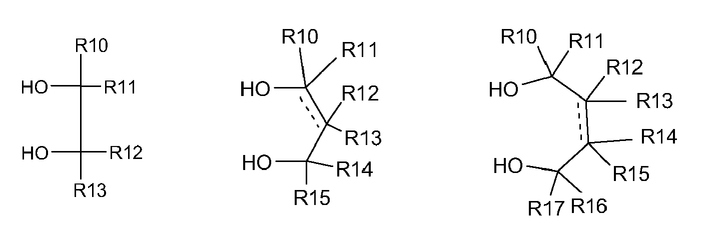 zu den cyclischen Acetalen der Formeln (Ib), (Ic) bzw. (Id)
unter Säurekatalyse und Wasserabscheidung umgesetzt wird,
wobei die Gruppen R1, R2, R3, R4, R5, R6, R7, R8, R9, R10, R11, R12, R13, R14, R15, R16 und R17 sowie die gestrichelten Linien jeweils die in Anspruch 1 angegebene Bedeutung haben.
